# EUROPÄISCHE PATENTSCHRIFT

(11) **EP 0 703 230 B1**
(45) Veröffentlichungstag und Bekanntmachung des Hinweises auf die Patenterteilung: **15.12.1999**
(21) Anmeldenummer: 95113754.6
(22) Anmeldetag: 01.09.1995
(51) Int. Cl.: C07D 319/06, C08G 64/02

(54) **1,3-Dioxan-2-on-Gruppen enthaltende Oligourethane**
Oligourethanes containing 1,3-dioxane-2-one groups
Oligouréthanes contenant des groupes 1,3-dioxane-2-one

(30) Priorität: 14.09.1994 DE 4432647
(43) Veröffentlichungstag der Anmeldung: 27.03.1996
(73) Patentinhaber: BAYER AG, 51368 Leverkusen (DE)
(72) Erfinder: Hovestadt, Wieland, Dr., D-47800 Krefeld (DE); Buysch, Hans-Josef, Dr., D-47809 Krefeld (DE); Schmalstieg, Lutz, Dr., D-50676 Köln (DE); Blum, Harald, Dr., D-47669 Wachtendonk (DE); Schön, Norbert, Dr., D-64289 Darmstadt (DE)

(56) Entgegenhaltungen:
- EP-A- 0 057 360
- MAKROMOLEKULARE CHEMIE, MACROMOLECULAR SYMPOSIA, Bd. 42/43, März 1991 BASEL CH, Seiten 145-153, H. HÖCKER ET AL. 'Ring-Opening Polymerization and Copolymerization of Cyclic Carbonates'

## Beschreibung

Die Erfindung betrifft ein Verfahren zur Herstellung von 1,3-Dioxan-2-on-Gruppen enthaltenden Oligourethanen, durch Umsetzung von hydroxyfunktionellen 1,3-Dioxan-2-onen a) mit organischen Polyisocyanaten, die nach diesem Verfahren erhältlichen Oligourethane und ihre Verwendung, gegebenenfalls in Kombination mit aktive Wasserstoffatome aufweisenden Verbindungen, zur Herstellung von Kunststoffen oder als Bindemittel bzw. Bindemittelkomponente in Beschichtungsmitteln.

Polyurethankunststoffe bzw. die zu Polyurethankunststoffen ausreagierenden Reaktionsgemische aus organischen Polyisocyanaten und organischen Polyhydroxylverbindungen haben aufgrund ihrer in weiten Grenzen variablen Produkteigenschaften eine breite Verwendung in fast allen Bereichen der Technik, insbesondere auch in der Beschichtungstechnologie gefunden. Zu den Nachteilen der Polyurethanchemie gehören die Feuchtigkeitsempfindlichkeit der eingesetzten Polyisocyanate, die bei der Herstellung von porenfreien Kunststoffen oder Beschichtungen oftmals besondere Vorkehrungen erforderlich macht, oder auch die Notwendigkeit des Einsatzes von Blockierungsmittel abspaltenden blockierten Polyisocyanaten bei der Herstellung von bei Raumtemperatur lagerstabilen Einbrennlacken.

Die der Erfindung zugrundeliegende Aufgabe bestand daher darin, auf der bekannten Polyurethanchemie aufbauend neue Kunststoffvorläufer zur Verfügung zu stellen, die aufgrund der Verwendbarkeit von Ausgangsmaterialien der unterschiedlichsten Beschaffenheit eine ebenso große Variabilität der Produkteigenschaften der letztendlich aus ihnen hergestellten Kunststoffe gestatten, ohne mit den aufgezeigten Nachteilen der Polyurethanchemie behaftet zu sein.

Diese Aufgabe konnte mit dem nachstehend näher beschriebenen erfindungsgemäßen Verfahren zur Herstellung von 1,3-Dioxan-2-on-Gruppen aufweisenden Oligourethanen gelöst werden. Die erfindungsgemäßen Verfahrensprodukte stellen Kunststoffvorläufer dar, die einerseits ohne Abspaltung von Blockierungsmitteln zu hochmolekularen Kunststoffen polymerisiert oder auch in Kombination mit aktive Wasserstoffatome aufweisenden Verbindungen der unterschiedlichsten Beschaffenheit zu hochmolekularen vernetzten Polyadditionsprodukten umgesetzt werden können, wobei die Variationsbreite der Eigenschaften der Endprodukte nicht nur auf die Variabilität der zuletzt genannten Reaktionspartner für die Oligourethane sondern auch auf die Variationsbreite der zur Herstellung der Oligourethane selbst eingesetzten Ausgangsmaterialien zurückzuführen ist.

Gegenstand der Erfindung ist ein Verfahren zur Herstellung von 1,3-Dioxan-2-on-Gruppen enthaltenden Oligourethanen, dadurch gekennzeichnet, daß man
a) hydroxyfunktionelle 1,3-Dioxan-2-one der allgemeinen Formel 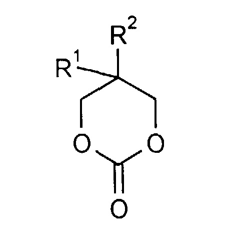 in welcher
   - R¹: einen Hydroxyalkylrest und R² einen Alkylrest bedeuten,
   mit
b) isocyanatfunktionellen Verbindungen, die im Mittel mindestens zwei Isocyanatgruppen pro Molekül aufweisen,
unter Urethanbildung umsetzt.

Gegenstand der Erfindung sind auch die nach diesem Verfahren erhältlichen Oligourethane.

Gegenstand der Erfindung ist auch die Verwendung der nach diesem Verfahren erhältlichen Oligourethane, gegebenenfalls in Kombination mit aktive Wasserstoffatome aufweisenden Verbindungen zur Herstellung von hochmolekularen Kunststoffen bzw. als Bindemittel oder Bindemittelkomponente in Beschichtungsmitteln.

Die erfindungsgemäß als Komponente a) einsetzbaren hydroxyfunktionellen 1,3-Dioxan-2-one sind bekannt und werden beispielhaft in EP-A 0 057 360 oder DE-OS 3 418 092 beschrieben. Als Komponente a) einsetzbar sind prinzipiell alle Verbindungen der allgemeinen Formel (I) 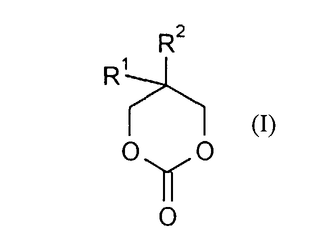 in welcher
- R¹: für einen Hydroxyalkylrest, vorzugsweise mit 1 - 10, insbesondere 1 - 4 Kohlenstoffatomen und
- R²: für einen Alkylrest, vorzugsweise mit 1 - 4, insbesondere 1 oder 2 Kohlenstoffatomen
stehen.

Besonders bevorzugt eingesetzt werden 1,3-Dioxan-5-hydroxymethyl-5-methyl-2-on oder 1,3-Dioxan-5-hydroxymethyl-5-ethyl-2-on.

Als Komponente b) kommen im Prinzip beliebige organische Verbindungen in Betracht, die pro Molekül mindestens 2 Isocyanatgruppen aufweisen. Gut geeignet sind vor allem organische Polyisocyanate, ausgewählt aus der Gruppe, bestehend aus (i) unmodifizierten organischen Polyisocyanaten des Molekulargewichtsbereichs 140 - 300, (ii) Lackpolyisocyanaten eines über 300 und bis 1 000 liegenden Molekulargewichts, (iii) Urethangruppen aufweisenden NCO-Prepolymeren eines über 1 000 liegenden Molekulargewichts und (iv) Gemischen der unter (i) bis (iii) genannten Polyisocyanate.

Beispiele für Polyisocyanate der Gruppe (i) sind 1,4-Diisocyanatobutan, 1,6-Diisocyanatohexan (HDI), 1,5-Diisocyanato-2,2-dimethylpentan, 2,2,4- bzw. 2,4,4-Trimethyl-1,6-diisocyanatohexan, 1-Isocyanato-3,3,5-trimethyl-5-isocyanatomethylcyclohexan (IPDI), 1-Isocyanto-1-methyl-4-(3)-isocyanatomethyl-cyclohexan, 1,3-Diisocyanato-6-methylcyclohexan, Bis-(4-isocyanatocyclohexyl)methan, 1,10-Diisocyanatodecan, 1,12-Diisocyanatododecan, Cyclohexan-1,3- und -1,4-diisocyanat, Xylylendiisocyanat-Isomere, 2,4-Diisocyanatotoluol oder dessen Gemische mit vorzugsweise bis zu 35 Gew.-%, bezogen auf Gemisch an 2,6-Diisocyanatotoluol, 2,2'-, 2,4'-, 4,4'-Diisocyanatodiphenylmethan oder technische Polyisocyanatgemische der Diphenylmethanreihe oder beliebige Gemische der genannten Isocyanate. Bevorzugte Polyisocyanate der Gruppe (i) sind HDI und IPDI.

Polyisocyanate der Gruppe (ii) sind die an sich bekannten Lackpolyisocyanate. Unter dem Begriff "Lackpolyisocyanate" sind im Rahmen der Erfindung Verbindungen bzw. Gemische von Verbindungen zu verstehen, die durch an sich bekannte Oligomerisierungsreaktion von einfachen Diisocyanaten der oben beispielhaft genannten Art erhalten werden.

Geeignete Oligomerisierungsreaktionen sind z.B. die Carbodiimidisierung, Dimerisierung, Trimerisierung, Biuretisierung, Harnstoffbildung, Urethanisierung, Allophanatisierung und/oder Cyclisierung unter Ausbildung von Oxadiazinstrukturen. Oftmals laufen bei der "Oligomerisierung" mehrere der genannten Reaktionen gleichzeitig oder nacheinander ab. Bevorzugt handelt es sich bei den "Lackpolyisocyanaten" um a) Biuretpolyisocyanate, b) Isocyanuratgruppen aufweisende Polyisocyanate, c) Isocyanurat- und Uretdiongruppen aufweisende Polyisocyanatgemische, d) Urethan- und/oder Allophanatgruppen aufweisende Polyisocyanate oder um e) Isocyanurat- und Allophanatgruppen aufweisende Polyisocyanatgemische auf Basis der oben genannten einfachen Diisocyanate, insbesondere auf Basis von HDI oder IPDI und besonders bevorzugt auf Basis von HDI.

Die Herstellung von derartigen Lackpolyisocyanaten ist bekannt und beispielsweise in DE-OS 1 595 273, DE-OS 3 700 209 und DE-OS 3 900 053 oder in EP-A-0 330 966, EP-A-0 259 233, EP-A-0 377 177, EP-A-0 496 208, EP-A-0 524 501 bzw. US-PS 4 385 171 beschrieben.

Polyisocyanate der Gruppe (iii) sind die an sich bekannten Isocyanatgruppen aufweisenden Prepolymere auf Basis von einfachen Diisocyanaten der unter (i) beispielhaft genannten Art und/oder auf Basis von Lackpolyisocyanaten der unter (ii) genannten Art einerseits und organischen Polyhydroxylverbindungen eines über 300 liegenden Molekulargewichts andererseits. Während es sich bei den Urethangruppen aufweisenden Lackpolyisocyanaten der Gruppe (ii) um Derivate von niedermolekularen Polyolen des Molekulargewichtsbereichs 62 bis 300 handelt (geeignete Polyole sind beispielsweise Ethylenglykol, Propylenglykol, Trimethylolpropan, Glycerin oder Gemische derartiger Alkohole), werden zur Herstellung der NCO-Prepolymeren der Gruppe (iii) Polyhydroxylverbindungen eines über 300, vorzugsweise über 500, besonders bevorzugt eines zwischen 500 und 4000 liegenden Molekulargewichts eingesetzt. Derartige Polyhydroxylverbindungen sind insbesondere solche, die pro Molekül 2 bis 6, vorzugsweise 2 bis 3 Hydroxylgruppen aufweisen und aus der Gruppe, bestehend aus Ether-, Ester-, Thioether-, Carbonat- und Polyacrylatpolyolen und Gemischen aus derartigen Polyolen ausgewählt sind.

Bei der Herstellung der NCO-Prepolymeren (iii) können die genannten höhermolekularen Polyole auch in Abmischungen mit den genannten niedermolekularen Polyolen zur Anwendung gelangen, so daß unmittelbar Gemische aus niedermolekularen, Urethangruppen aufweisenden Lackpolyisocyanaten (ii) und höhermolekularen NCO-Prepolymeren (iii) resultieren, die ebenfalls als erfindungsgemäße Ausgangskomponente b) geeignet sind.

Zur Herstellung der NCO-Prepolymeren (iii) bzw. ihrer Gemische mit den Lackpolyisocyanaten (ii) werden Diisocyanate der unter (i) beispielhaft geannnten Art oder Lackpolyisocyanate der unter (iii) beispielhaft genannten Art mit den höhermolekularen Hydroxylverbindungen bzw. deren Gemischen mit niedermolekularen Polyhydroxylverbindungen der beispieslhaft genannten Art unter Einhaltung eines NCO/OH-Äquivalentverhältnisses von 1,1:1 bis 40:1, vorzugsweise 2:1 bis 25:1 unter Urethanbildung umgesetzt. Gewünschtenfalls kann bei Verwendung eines Überschusses an destillierbarem Ausgangsdiissocyanat (i) dieser Überschuß im Anschluß an die Umsetzung destillativ erntfernt werden, so daß monomerenfreie NCO-Prepolymere vorliegen. Falls als Isocyanatkomponente Diisocyanate der unter (i) beispielhaft genannten Art im Überschuß eingesetzt worden sind und auf die destillative Entfernung des nicht umgesetzten Überschusses verzichtet wird, liegen NCO-Semiprepolymere, d.h. Gemische aus Ausgangsdiisocyanaten der Gruppe (i) und echten NCO-Prepolymeren (iii) vor, die ebenfalls als erfindungsgemäße Ausgangskomponente b) eingesetzt werden können.

Durch geeignete Wahl der Art der beim erfindungsgemäßen Verfahren einzusetztenden Komponente b) können die Eigenschaften der erfindungsgemäßen Verfahrensprodukte dem gewünschten Einsatzzweck angepaßt werden. Eine weitere Variationsmöglichkeit der aus den erfindungsgemäßen Verfahrensprodukten letztendlich erhaltenen hochmolekularen Kunststoffe bzw. Beschichtungen besteht in der geeigneten Auswahl der als Reaktionspartner für die erfindungsgemäßen Oligourethane eingesetzten Verbindungen mit aktiven Wasserstoffatomen.

Beim erfindungsgemäßen Verfahren kommen die erfindungswesentlichen Ausgangskomponenten a) und b) in solchen Mengen zum Einsatz, die einem OH/NCO-Äquivalentverhältnis von 0,5:1 bis 2:1, vorzugsweise 0,9:1 bis 1,1:1, besonders bevorzugt 1:1 entsprechen.

Die erfindungsgemäße Umsetzung wird entweder lösemittelfrei oder in einem geeigneten aprotischen Lösemittel oder Lösemittelgemisch, wie beispielsweise Toluol, Xylol, Butylacetat, Ethylacetat, Ethylglykolacetat, Pentylacetat, Hexylacetat, Methoxypropylacetat, Tetrahydrofuran, Dioxan, Dibutyldiglykol, Aceton, Methylethylketon, Methylisobutylketon, Cyclopentanon, Cyclohexanon, N-Methylpyrrolidon, Dimethylformamid, höher substituierte Aromaten, wie z.B. Solventnaphtha, Schwerbenzol, verschiedene ®Solvesso-Typen und ®Diasol sowie höhersiedende aliphatische und cycloaliphatische Kohlenwasserstoffe, wie z.B. verschiedene Testbenzine, Mineralterpentinöl, ®Ispar-Typen, ®Nappar-Typen, Tetralin und Dekalin durchgeführt.

Die erfindungsgemäße Umsetzung wird im allgemeinen innerhalb des Temperaturbereiches von 20 - 130°C, vorzugsweise 40 - 100°C, durchgeführt. Die Menge des Lösungsmittels entspricht hierbei im allgemeinen einer Konzentration der Ausgangskomponenten a) und b) von 0 - 80, vorzugsweise 0 - 50 Gew.-%.

Zur Beschleunigung der Umsetzung der Komponente a) und b) können gegebenenfalls Katalysatoren wie beispielsweise Triethylamin, Tributylamin, 1,4-Diazabicyclo-(2,2,2)-octan, N,N-Dimethylbenzylamin, 2-Methylimidazol, Pyridin, Mannichbasen, Tetraalkylammoniumhydroxide und Alkalihydroxide wie Lithiumhydroxid, Natriumhydroxid, Alkaliphenolate, Metallsalze wie Eisen-(III)-chlorid, Zinnverbindungen wie Zinn-(II)-acetat, Zinn-(II)-octoat, Zinn-(II)-ethylhexanoat, Zinn-(II)-laurat, Dibutylzinnoxid, Dibutylzinndichlorid, Dibutylzinndiacetat, Dibutylzinndilaurat, Dibutylzinnmaleat oder Dioctylzinndiaceat eingesetzt werden.

Die erfindungsgemäßen 1,3-Dioxan-2-on-Gruppen enthaltenden Oligourethane eignen sich zur Herstellung von Kunststoffen oder Beschichtungsmaterialien. Sie werden entweder als Einkomponenten-Einbrennsystemen oder in Kombination mit geeigneten Härtern als Zweikomponentensystemen verarbeitet.

Bei der Verwendung als Einkomponenten-Einbrennsystem können alle Katalysatoren zugesetzt werden, die als Katalysatoren für die ringöffnende Polymerisation von 1,3-Dioxan-2-onen bekannt sind (vgl. z.B. EP-A-0 209 722, EP-A-0 188 204, DE-OS 1 545 116, DE-OS 1 545 117, DE-OS 1 545 118 oder JACS 52 (1930) 314. Es sind dies beispielsweise die oben bereits beispielhaft genannten Katalysatoren oder aber auch beispielsweise Umsetzungsprodukte von Alkylphosph0aten oder -phosphonaten mit Phosphinen, Aminen, Amiden oder N-heterocyclischen Verbindungen, Alkalialkoholate, Alkalicarboxylate, Säureamide, Harnstoffe oder Amidine.

Die Aushärtung der erfindungsgemäßen Oligourethane bei ihrer Verwendung als Bindemittel in Einbrennlacken erfolgt innerhalb des Temperaturbereichs von 80 bis 180°C.

Bei der erfindungsgemäßen Verwendung der erfindungsgemäßen Oligourethane in Zweikomponentensystemen können beliebige Verbindungen mit mindestens zwei Gruppen mit Zerewitinow-aktiven Wasserstoffatomen eingesetzt werden. Geeignet sind beispielsweise Polycarbonsäuren, Hydroxycarbonsäure, Polyanhydride, Polyhydroxylverbindungen, Polyamine, Aminoalkohole, Aminosäuren oder Polyurethane mit mindestens zwei funktionellen Gruppen, wobei sowohl niederals auch höhermolekulare Verbindungen der beispielhaft genannten Art in Betracht kommen. Grundsätzlich möglich ist auch die Verwendung von Verbindungen mit potentiellen, beispielsweise durch Feuchtigkeitseinfluß aktivierbaren H-aktiven Gruppen, wie beispielsweise von Bisketiminen, Bisaldiminen oder Polyoxazolidinen. Die beispielhaft genannten Reaktionspartner können bei der erfindungsgemäßen Verwendung der erfindungsgemäßen Oligourethane in Zweikomponenten-Systemen in, bezüglich der Dioxanon-Grupen, etwa äquivalenten Mengen aber auch in stark unterschüssigen Mengen eingesetzt werden. Je nach Reaktivität erfolgt die Aushärtung durch Addition des Härters unter Ringöffnung der 1,3-Dioxan-2-on-Gruppen bei Temperaturen zwischen 0 und 180°C.

Bei der erfindungsgemäßen Verwendung der erfindungsgemäßen Oligourethane als Bindemittel bzw. Bindemittel komponenten in Beschichtungsmitteln können letzteren selbstverständlich die üblichen Hilfs- und Zusatzmittel einverleibt werden. Hierzu gehören beispielsweise Verlaufsmittel, z.B. auf Celluloseester- oder Oligoacrylatbasis, Pigmente und Füllstoffe, viskositätskontrollierende Zusätze wie Bentonite und Kieselsäureester, Mattierungsmittel wie beispielsweise Kieselsäure, Aluminiumsilikate und hochmolekulare Wachse oder Katalysatoren für die Vernetzungsreaktion, wie beispielsweise Zinn(II)-octoat, Dibutylzinnoxid, Triethylamin oder Natriumhydroxid.

Die folgenden Beispiele sollen die Erfindung näher erläutern, ohne sie jedoch einzuschränken. Alle Angaben in "%" beziehen sich auf das Gewicht.

### Beispiele

### Beispiel 1

### Herstellung von 1,3-Dioxan-5-hydromethyl-5-ethyl-2-on

1,608 kg (12 Mol) Trimethylolpropan, 1,416kg (12 Mol) Diethylcarbonat und 300 mg pulvriges KOH werden unter Rühren an einer 1,2 m-Füllkörperkolonne während 4 Stunden auf 110 bis 130°C Innentemperatur erhitzt. Über Kopf werden 1 065 g Ethanol abdestilliert, wobei gegen Ende der Umesterung das Ethanol durch stufenweuse Verringerung des Druckes bis auf 25 mbar entfernt wird. Das Rohprodukt wird bei einer Innentemperatur von 100°C mit 924 mg p-Toluolsulfonsäure versetzt und 30 Minuten bei dieser Temperatur gerührt. Das so erhaltene Polycarbonat wird durch Eintropfen aus einem auf 150°C beheizten Tropftrichter in einem auf 220°C vorgeheizten Kolben, in den 1,5 g Zinnschliff vorgelegt werden, unter Rühren bei einem Druck von 0,05 mbar depolymerisiert und die entstehenden Dämpfe in hintereinander angeordneten Kühlfallen (mit Methanol/Trockeneis bzw. flüssigem Stickstoff gekühlt) kondensiert. Es werden 1 730 g (90 % der Theorie) farbloses Kondensat erhalten, das bei Raumtemperatur kristallisiert.

### Beispiel 2

In einem Dreihalskolben mit Rührer, Tropftrichter und Thermometer werden 222 g (1 Mol) 1-Isocyanato-3,3,5-trimethyl-5-isocyanatomethylcyclohexan (IPDI) und 0,1 Dibutylzinnoxid in 200 g Butylacetat gelöst vorgelegt und auf 70°C erwärmt. Zu dieser Lösung werden innerhalb von 1 Stunde 324 g (2 Mol) 1,3-Dioxan-5-hydroxymethyl-5-ethyl-2-on aus Beispiel 1, gelöst in 200 g Butylacetat, gegeben. Nach 5 Stunden ist die Reaktion abgeschlossen. Es resultiert eine Lösung eines erfindungsgemäßen Oligourethans mit 2-Dioxanon-Gruppen pro Molekül.

### Beispiel 3

In einem Dreihalskolben mit Rührer, Tropftrichter und Thermometer werden 100 g N-Methylpyrrolidon, 100 g (0,62 Mol) 1,3-Dioxan-5-hydromethyl-5-ethyl-2-on aus Beispiel 1 und 122 g eines Isocyanuratgruppen aufweisenden Lackpolyisocyanats auf HDI-Basis mit einem NCO-Gehalt von 21,8 % (®Desmodur N 3300 der Bayer AG) zur Umsetzung gebracht. Nach 8 Stunden bei 70°C ist die Umsetzung abgeschlossen. Es resultiert ein erfindungsgemäßes Oligourethan mit einer über drei liegenden Funktionalität bezüglich der Dioxanon-Gruppen.

### Beispiel 4

In einem Dreihalskolben mit Rührer, Tropftrichter und Thermometer werden 81 g (0,50 Mol) 1,3-Dioxan-5-hydroxymethyl-5-ethyl-2-on aus Beispiel 1 und 200 g (entsprechend 0,50 Mol NCO) ®Desmodur 3265 (aliphatisches Polyisocyanat-Prepolymer der Bayer AG auf Basis eines Polyesterpolyols und ®Desmodur N 3300, 65 %ig gelöst in Solventnaphtha/Methoxypropylacetat (3,8: 1) mit einem NCO-Gehalt der Lösung von 10,4 %), eingewogen auf 70°C erwärmt. Nach 7 Stunden ist die Reaktion abgeschlossen. Es resultiert ein erfindungsgemäßes Oligourethan mit einer über zwei liegenden Funktionalität bezüglich der 1,3-Dioxanon-Gruppen.

### Beispiel 5

100 g des Produktes aus Beispiel 2 werden mit 100 g ®Desmophen A 450, einem hydroxyfunktionellen Polyacrylat der Bayer AG, 50 % in Butylacetat mit einem OH-Gehalt der Lösung von 1,1 %, versetzt. Die Abmischung ist lagerstabil. Sie wurde auf ein Prüfblech appliziert. Innerhalb von 30 Minuten bei 155°C resultiert ein harter, lösemittelbeständiger Lackfilm.

### Beispiel 6

100 g des Produktes aus Beispiel 3 werden mit 17 g N,N'-Dimethylethylendiamin versetzt und auf ein Prüfblech appliziert. Innerhalb von 30 Minuten bei 80°C resultiert ein harter, lösemittelbeständiger Lackstoff.

### Beispiel 7

100 g des Produktes aus Beispiel 4 werden mit 20 g Hexamethylendiamin in 50 g Methoxypropylacetat versetzt und auf ein Prüfblech appliziert. Innerhalb von 30 Minuten bei 80°C resultiert ein harter, lösemittelbeständiger Lackstoff.

### Beispiel 8

100 g des Produktes aus Beispiel 3 werden mit 1 einer 3 %igen Lösung von Kalium-tert.-butylat in ε-Caprolactam versetzt und auf ein Prüfblech appliziert. Innerhalb von 30 Minuten bei 160°C resultiert ein lösemittelbeständiger ausgehärteter Lackfilm.

## Patentansprüche

1. Verfahren zur Herstellung von 1,3-Dioxan-2-on-Gruppen enthaltenden Oligourethanen, dadurch gekennzeichnet, daß man
a) hydroxyfunktionelle 1,3-Dioxan-2-one der allgemeinen Formel 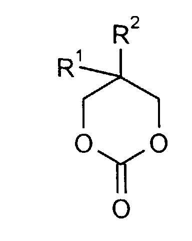 in welcher
R¹ einen Hydroxyalkylrest und R² einen Alkylrest bedeuten,
mit
b) isocyanatfunktionellen Verbindungen, die im Mittel mindestens zwei Isocyanatgruppen pro Molekül aufweisen,
unter Urethanbildung umsetzt.

2. Verfahren gemäß Anspruch 1, dadurch gekennzeichnet, daß man als Komponente a) 1,3-Dioxan-2-one der in Anspruch 1 genannten Formel verwendet,
für welche
R¹ einen C₁-C₄-Hydroxyalkylrest und
R² einen C₁-C₄-Alkylrest bedeuten.

3. Verfahren gemäß Anspruch 1 und 2, dadurch gekennzeichnet, daß man als Komponente a) 1,3-Dioxan-5-hydroxymethyl-5-methyl-2-on oder 1,3-Dioxan-5-hydromethyl-5-ethyl-2-on verwendet.

4. Verfahren gemäß Anspruch 1 bis 3, dadurch gekennzeichnet, daß man als Komponente b) organische Polyisocyanate ausgewählt aus der Gruppe bestehend aus (i) unmodifizierten organischen Polyisocyanaten des Molekulargewichtsbereichs 140 - 300, (ii) Lackpolyisocyanaten eines über 300 und bis 1 000 liegenden Molekulargewichts, (iii) Urethangruppen aufweisenden NCO-Prepolymeren eines über 1 000 liegenden Molekulargewichts und (iv) Gemischen der vorstehend genannten Polyisocyante, verwendet.

5. Gemäß Anspruch 1 bis 4 erhältliche Oligourethane.

6. Verwendung der gemäß Anspruch 1 bis 4 erhältlichen Oligourethane, gegebenenfalls in Kombination mit aktive Wassserstoffatome aufweisenden Verbindungen zur Herstellung von Kunststoffen.

7. Verwendung der gemäß Anspruch 1 bis 4 erhältlichen Oligourethane, gegebenenfalls in Kombination mit aktive Wasserstoffatome aufweisenden Verbindungen als Bindemittel bzw. Bindemittelkomponente in Beschichtungsmitteln.

## Claims

1. A process for the preparation of 1,3-dioxan-2-one group-containing oligourethanes, characterised in that
a) hydroxy-functional 1,3-dioxan-2-ones of the general formula 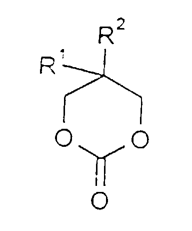 in which
R¹ denotes a hydroxyalkyl radical and R² an alkyl radical
are reacted with
b) isocyanate-functional compounds which exhibit on average at least two isocyanate groups per molecule,
with formation of urethane.

2. A process according to Claim 1, characterised in that 1,3-dioxan-2-ones of the formula indicated in Claim 1 are used as component a),
in which
R¹ denotes a C₁-C₄ hydroxyalkyl radical, and
R² stands for a C₁-C₄ alkyl radical.

3. A process according to Claims 1 and 2, characterised in that 1,3-dioxan-5-hydroxymethyl-5-methyl-2-one or 1,3-dioxan-5-hydroxymethyl-5-ethyl-2-one are used as component a).

4. A process according to Claims 1 to 3, characterised in that organic polyisocyanates selected from the group comprising (i) unmodified organic polyisocyanates within the molecular weight range 140 to 300, (ii) lacquer polyisocyanates of a molecular weight greater than 300 and up to 1,000, (iii) urethane group-exhibiting NCO prepolymers of a molecular weight greater than 1,000 and (iv) mixtures of the aforementioned polyisocyanates are used as component b).

5. Oligourethanes obtainable according to Claims 1 to 4.

6. Use of the oligourethanes obtainable according to Claims 1 to 4, optionally in combination with compounds exhibiting active hydrogen atoms, for the preparation of plastics.

7. Use of the oligourethanes obtainable according to Claims 1 to 4, optionally in combination with compounds exhibiting active hydrogen atoms, as a binder or binder component in coating agents.

## Revendications

1. Procédé de préparation d'oligo-uréthannes contenant des groupes 1,3-dioxanne-2-one, caractérisé en ce que l'on fait réagir, avec formation d'uréthannes
a) des 1,3-dioxanne-2-ones à fonctions hydroxy de formule générale 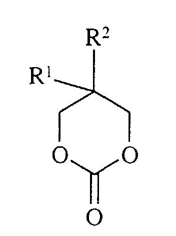 dans laquelle
R¹ représente un groupe hydroxyalkyle et R² un groupe alkyle,
avec
b) des composés à fonctions isocyanate contenant en moyenne au moins deux groupes isocyanate par molécule.

2. Procédé selon la revendication 1, caractérisé en ce que l'on utilise en tant que composants a) des 1,3-dioxanne-2-ones répondant à la formule donnée dans la revendication 1, dans laquelle
R¹ représente un groupe hydroxyalkyle en C₁-C₄ et
R² un groupe alkyle en C₁-C₄.

3. Procédé selon les revendications 1 et 2, caractérisé en ce que l'on utilise en tant que composant a) la 1,3-dioxanne-5-hydroxyméthyl-5-méthyl-2-one ou la 1,3-dioxanne-5-hydrométhyl-5-éthyl-2-one.

4. Procédé selon les revendications 1 à 3, caractérisé en ce que l'on utilise en tant que composants b) des polyisocyanates organiques choisis dans le groupe consistant en (i) les polyisocyanates organiques non modifiés de poids moléculaire 140 à 300, (ii) les polyisocyanates pour vernis de poids moléculaire supérieur à 300 et pouvant aller jusqu'à 1 000, (iii) les prépolymères à groupes NCO, contenant également des groupes uréthanne, de poids moléculaire supérieur à 1 000, et (iv) les mélanges de ces polyisocyanates.

5. Les oligo-uréthannes obtenus selon les revendications 1 à 4.

6. Utilisation des oligo-uréthannes obtenus selon les revendications 1 à4, le cas échéant en combinaison avec des composés portant des atomes d'hydrogène actifs, pour la préparation de résines synthétiques.

7. Utilisation des oligo-uréthannes obtenus selon les revendications 1 à4, éventuellement en combinaison avec des composés portant des atomes d'hydrogène actifs, en tant que liants ou composants de liants de produits de revêtement.
